Europäisches Patentamt

European Patent Office

Office européen des brevets

(19)

(11) **EP 1 178 810 B1**

(12) **EUROPEAN PATENT SPECIFICATION**

(45) Date of publication and mention
of the grant of the patent:
**20.04.2005 Bulletin 2005/16**

(51) Int Cl.7: **A61K 31/661**, A61P 35/00,
A61P 9/00

(21) Application number: **00936760.8**

(22) Date of filing: **19.05.2000**

(86) International application number:
**PCT/EP2000/004562**

(87) International publication number:
**WO 2000/071104 (30.11.2000 Gazette 2000/48)**

(54) **USE OF BISPHOSPHONIC ACIDS FOR TREATING ANGIOGENESIS**

VERWENDUNG VON BIPHOSPHONSÄUREN ZUR BEHANDLUNG VON ANGIOGENESE

UTILISATION D'ACIDE BIPHOSPHONIQUE POUR TRAITER L'ANGIOGENESE

(84) Designated Contracting States:
**AT BE CH CY DE DK ES FI FR GB GR IE IT LI LU MC NL PT SE**
Designated Extension States:
**SI**

(30) Priority: **21.05.1999 GB 9911926**
**22.10.1999 GB 9925131**

(43) Date of publication of application:
**13.02.2002 Bulletin 2002/07**

(73) Proprietors:
• **Novartis AG**
**4056 Basel (CH)**
Designated Contracting States:
**BE CH DE DK ES FI FR GB GR IE IT LI LU MC NL PT CY**
• **Novartis Pharma GmbH**
**1230 Wien (AT)**
Designated Contracting States:
**AT**

(72) Inventors:
• **OKUNO, Tetsuji**
**Saitama Prefecture 360-0805 (JP)**
• **GREEN, Jonathan**
**CH-4144 Arlesheim (CH)**
• **WOOD, Jeanette, Marjorie**
**CH-4105 Biel-Benken (CH)**

(74) Representative: **de Weerd, Petrus G.W. et al**
**Novartis International AG**
**Corporate Intellectual Property**
**4002 Basel (CH)**

(56) References cited:
**EP-A- 0 998 934**     **WO-A-88/00829**
**WO-A-95/29679**     **WO-A-98/43987**
**WO-A-99/04773**     **WO-A-99/38998**
**US-A- 4 608 368**     **US-A- 5 358 941**
**US-A- 5 866 556**

• **SHIPMAN: "antitumor activity..." LEUK. LYMPHOMA, vol. 32, no. 1/2, 1998, pages 129-138, XP000980146**
• **MARTODAM: "the effects of dichloroethylene..." CALCIF. TISSUE INT., vol. 35, no. 4-5, 1983, pages 512-519, XP000980229**

**Description**

This invention relates to new therapeutic uses of bisphosphonates.

Bisphosphonates are widely used to inhibit osteoclast activity in a variety of both benign and malignant diseases in which bone resorption is increased. Thus bisphosphonates have recently become available for long-term treatment of patients with Multiple Myeloma (MM). These pyrophosphate analogs not only reduce the occurrence of skeletal related events but they also provide patients with clinical benefit and improve survival. Bisphosphonates are able to prevent bone resorption *in vivo*; the therapeutic efficacy of bisphosphonates has been demonstrated in the treatment of Paget's disease of bone, tumour-induced hypercalcemia and, more recently, bone metastasis and multiple myeloma (MM) (for review see Fleisch H 1997 Bisphosphonates clinical. In Bisphosphonates in Bone Disease. From the Laboratory to the Patient. Eds: The Parthenon Publishing Group, New York/London pp 68-163). The mechanisms by which bisphosphonates inhibit bone resorption are still poorly understood and seem to vary according to the bisphosphonates studied. Bisphosphonates have been shown to bind strongly to the hydroxyapatite crystals of bone, to reduce bone turn-over and resorption, to decrease the levels of hydroxyproline or alkaline phosphatase in the blood, and in addition to inhibit both the activation and the activity of osteoclasts.

MM is a plasma-cell malignancy characterized by the proliferation and the accumulation of malignant plasma cells within the bone marrow. The main clinical consequences are lytic bone lesions associated with pathologic fractures and bone pain. These lesions result from an excessive bone resorption, frequently leading to hypercalcemia. Bisphosphonates have been introduced for the long-term treatment of MM in combination with conventional chemotherapy. It has been shown recently that bisphosphonates such as clodronate and pamidronate can reduce the occurrence of skeletal related events such as lytic bone lesions and pathologic fractures and can relieve bone pain and improve the quality of life of patients.

It has now been surprisingly found that certain bisphosphonates have an embolic effect on the newly formed capillary blood vessels which form during angiogenesis associated with tumour growth and invasion and certain other pathological conditions such as inflammation, rheumatoid arthritis and osteoarthritis. Furthermore it has been found that certain bisphosponates inhibit growth factor induced angiogenesis and endothelial cell proliferation in animal model and tissue culture experiments.

Accordingly the present invention provides use of a bisphosphonate in the preparation of a medicament for the treatment of myocardial ischemia, osteoarthritis, psoriasis, haemangioblastoma, haemangioma or pain, wherein the bisphosphonate acts as an angiogenesis inhibiting or reversing agent and is selected from the following compounds or a pharmaceutically acceptable salt thereof, or any hydrate thereof: 3-amino-1-hydroxypropane-1,1-diphosphonic acid (pamidronic acid), e.g. pamidronate (APD); 3-(N,N-dimethylamino)-1-hydroxypropane-1,1-diphosphonic acid, e. g. dimethyl-APD; 4-amino-1-hydroxybutane-1,1-diphosphonic acid (alendronic acid), e.g. alendronate; 1-hydroxy-ethidene-bisphosphonic acid, e.g. etidronate; 1-hydroxy-3-(methylpentylamino)-propylidene-bisphosphonic acid, ibandronic acid, e.g. ibandronate; 6-amino-1-hydroxyhexane-1,1-diphosphonic acid, e.g. amino-hexyl-BP; 3-(N-methyl-N-n-pentylamino)-1-hydroxypropane-1,1-diphosphonic acid, e.g. methyl-pentyl-APD (= BM 21.0955); 1-hydroxy-2-(imidazol-1-yl)ethane-1,1-diphosphonic acid, e.g. zoledronic acid; 1-hydroxy-2-(3-pyridyl)ethane-1,1-diphosphonic acid (risedronic acid), e.g. risedronate, including N-methyl pyridinium salts thereof, for example N-methyl pyridinium iodides such as NE-10244 or NE-10446; 1-(4-chlorophenylthio)methane-1,1-diphosphonic acid (tiludronic acid), e.g. tiludronate; 3-[N-(2-phenylthioethyl)-N-methylamino]-1-hydroxypropane-1,1-diphosphonic acid; 1-hydroxy-3-(pyrrolidin-1-yl) propane-1,1-diphosphonic acid, e.g. EB 1053 (Leo); 1-(N-phenylaminothiocarbonyl)methane-1,1-diphosphonic acid, e.g. FR 78844 (Fujisawa); 5-benzoyl-3,4-dihydro-2H-pyrazole-3,3-diphosphonic acid tetraethyl ester, e.g. U-81581 (Upjohn); 1-hydroxy-2-(imidazo[1,2-a]pyridin-3-yl)ethane-1,1-diphosphonic acid, e.g. YM 529; and 1,1-dichloromethane-1,1-diphosphonic acid (clodronic acid), e.g. clodronate.

The uses of the present invention represent an improvement to existing therapy of malignant diseases in which bisphosphonates are used to prevent or inhibit development of bone metastases or excessive bone resorption, and also for the therapy of inflammatory diseases such as rheumatoid arthritis and osteoarthritis. Use of bisphosphonates to embolise newly formed blood vessels has been found to lead to suppression of tumours, e.g. solid tumours, and metastastes, e.g. bone metastases and even reduction in size of tumours, e.g. solid tumours, and metastases, e. g. bone metastases, after appropriate periods of treatment. It has been observed using angiography that newly formed blood vessels disappear after bisphosphonate treatment, but that normal blood vessels remain intact. Further it has been observed that the embolised blood vessels are not restored following cessation of the bisphosphonate treatment. Also it has been observed that bone metastasis, rheumatoid arthritis and osteoarthritis patients experience decreased pain following bisphosphonate treatment.

Although the mode of action of bisphosphonates as agents which cause embolism of newly formed blood vessels is not known, it appears that the newly formed blood vessels (capillaries) become blocked, partially or completely obliterated or angiogenesis is otherwise reversed, leading to a partial or complete disappearance of the newly formed blood vessels (capillaries), for instance, when the tumour or disease site, e.g. site of inflammation, is viewed using angiography. For the purposes of the present description the terms "embolic treatment of angiogenesis" or "embolic effect" refer to these observed phenomena.

Accordingly in a further aspect the invention provides use of a bisphosphonate in the preparation of a medicament as defined above for the embolic treatment of angiogenesis.

In addition as hereinafter described in the Examples certain bisphosphonates have been found to inhibit growth factor induced angiogenesis in an animal model and also to inhibit endothelial cell proliferation in a tissue culture model. It appears that such inhibition of angiogenesis is not dependent upon depletion of activated macrophages but rather that the bisphosphonate appears to act at the level of endothelial cell activation and/or endothelial cell proliferation. Thus, advantageously bisphosphonates may be used also for prophylactic or preventive treatment of diseases and medical conditions which involve angiogenesis, by inhibiting the occurrence or development of angiogenesis, including diseases and medical conditions as identified above.

Accordingly in a yet further aspect the invention provides:

- use of a bisphosphonate in the preparation of a medicament as defined above for the prophylactic or preventative treatment of angiogenesis.

In particular the invention provides a use as defined immediately above which does not dependent upon or involve depletion of activated macrophages.

Thus in the present description the terms "treatment" or "treat" refer to both prophylactic or preventative treatment as well as curative or disease modifying treatment, including treatment of patients at risk of contracting the disease or suspected to have contracted the disease as well as patients who are ill or have been diagnosed as suffering from a disease or medical condition.

The bisphosphonates used in the present invention are as defined above.

Pharmaceutically acceptable salts of the bisphosphonates for use in the invention are preferably salts with bases, conveniently metal salts derived from groups Ia, Ib, IIa and IIb of the Periodic Table of the Elements, including alkali metal salts, e.g. potassium and especially sodium salts, or alkaline earth metal salts, preferably calcium or magnesium salts, and also ammonium salts with ammonia or organic amines.

Especially preferred pharmaceutically acceptable salts are those where one, two, three or four, in particular one or two, of the acidic hydrogens of the bisphosphonic acid are replaced by a pharmaceutically acceptable cation, in particular sodium, potassium or ammonium, in first instance sodium.

A very preferred group of pharmaceutically acceptable salts is characterized by having one acidic hydrogen and one pharmaceutically acceptable cation, especially sodium, in each of the phosphonic acid groups.

All the bisphosphonic acid derivatives mentioned above are well known from the literature. This includes their manufacture (see e.g. EP-A-513760, pp. 13-48). For example, 3-amino-1-hydroxypropane-1,1-diphosphonic acid is prepared as described e.g. in US patent 3,962,432 as well as the disodium salt as in US patents 4,639,338 and 4,711,880, and 1-hydroxy-2-(imidazol-1-yl)ethane-1,1-diphosphonic acid is prepared as described e.g. in US patent 4,939,130.

A particular embodiment of the invention is represented by the use of a bisphosphonic acid derivative which is selected from 3-amino-1-hydroxypropane-1,1-diphosphonic acid, 3-(N,N-di-methylamino)-1-hydroxypropane-1,1-diphosphonic acid; 4-amino-1-hydroxybutane-1,1-diphosphonic acid; 6-amino-1-hydroxyhexane-1,1-diphosphonic acid, 3-(N-methyl-N-n-pentylamino)-1-hydroxypropane-1,1-diphosphonic acid; 1-hydroxy-2-(imidazol-1-yl)ethane-1,1-diphosphonic acid; 1-hydroxy-2-(3-pyridyl)ethane-1,1-diphosphonic acid, and N-methyl pyridinium salts thereof; 1-(4-chlorophenylthio)methane-1,1-diphosphonic acid; 3-[N-(2-phenylthioethyl)-N-methylamino]-1-hydroxypropane-1,1-diphosphonic acid; 1-hydroxy-3-(pyrrolidin-1-yl)propane-1,1-diphosphonic acid; 1-(N-phenylaminothiocarbonyl)methane-1,1-diphosphonic acid; 5-benzoyl-3,4-dihydro-2H-pyrazole-3,3-diphosphonic acid tetraethyl ester; 1-hydroxy-2-(imidazo[1,2-a]pyridin-3-yl)ethane-1,1-diphosphonic acid; or a pharmaceutically acceptable salt thereof, and any hydrate thereof.

A preferred embodiment of the invention is represented by the use of a bisphosphonic acid derivative which is selected from 3-amino-1-hydroxypropane-1,1-diphosphonic acid; 3-(N,N-di-methylamino)-1-hydroxypropane-1,1-diphosphonic acid; 4-amino-1-hydroxybutane-1,1-diphosphonic acid; 6-amino-1-hydroxyhexane-1,1-diphosphonic acid; 3-(N-methyl-N-n-pentylamino)-1-hydroxypropane-1,1-diphosphonic acid, 1-hydroxy-2-(imidazol-1-yl)ethane-1,1-diphosphonic acid; 1-hydroxy-2-(3-pyridyl)ethane-1,1-diphosphonic acid; 3-[N-(2-phenylthioethyl)-N-methylamino]-1-hydroxypropane-1,1-diphosphonic acid; 1-hydroxy-3-(pyrrolidin-1-yl)-propane-1,1-diphosphonic acid; 1-hydroxy-2-(imidazo[1,2-a]pyridin-3-yl)ethane-1,1-diphosphonic acid; or a pharmaceutically acceptable salt thereof, and any hydrate thereof.

A very preferred embodiment of the invention is represented by the use of a phosphonic acid derivative which is selected from pamidronic acid, alendronic acid, 3-(N-methyl-N-n-pentylamino)-1-hydroxypropane-1,1-diphosphonic acid; 1-hydroxy-2-(imidazol-1-yl)ethane-1,1-diphosphonic acid; risedronic acid and tiludronic acid; or a pharmaceutically acceptable salt thereof, and any hydrate thereof.

An especially preferred embodiment of the invention is represented by the use of a bisphosphonic acid derivative which is selected from 1-hydroxy-2-(imidazol-1-yl)ethane-1,1-diphosphonic acid and 3-amino-1-hydroxypropane-1,1-diphosphonic acid, or a pharmaceutically acceptable salt thereof, and any hydrate thereof.

Further the invention relates to the use of 3-amino-1-hydroxypropane-1,1-diphosphonic acid or a pharmaceutically acceptable salt thereof or any hydrate thereof, e.g. pamidronate disodium or pamidronate.

Further the invention relates to the use of 1-hydroxy-2-(imidazol-1-yl)ethane-1,1-diphosphonic acid or a pharmaceutically acceptable salt thereof or any hydrate thereof, e.g. zoledronic acid.

It has been found in an animal model in accordance with the present invention that zoledronic acid (1-hydroxy-2-(imidazol-1-yl)ethane-1,1-diphosphonic acid) preferentially inhibits basic fibroblast growth factor (bFGF) induced angiogenesis as compared with its inhibition of vascular endothelial growth factor (VEGF) induced angiogenesis, as hereinafter described in the Examples.

Accordingly in particularly preferred embodiments the invention provides a use as defined above

- in which the bishposphonate is zoledronic acid or a pharmaceutically acceptable salt thereof or any hydrate thereof and in which the angiogenesis comprises bFGF-induced angiogenesis, or
- in which the bisphosphonate is zoledronic acid or a pharmaceutically acceptable salt thereof or any hydrate thereof and the bisphosphonate is used in combination with a VEGF inhibitor.

The bisphosphonates (hereinafter referred to as the Agents of the Invention) may be used in the form of an isomer or of a mixture of isomers where appropriate, typically as optical isomers such as enantiomers or diastereoisomers or geometric isomers, typically cis-trans isomers. The optical isomers are obtained in the form of the pure antipodes and/or as racemates.

The Agents of the Invention can also be used in the form of their hydrates or include other solvents used for their crystallisation.

The Agents of the Invention (the bisphosphonates) are preferably used in the form of pharmaceutical compositions that contain a therapeutically effective amount of active ingredient optionally together with or in admixture with inorganic or organic, solid or liquid, pharmaceutically acceptable carriers which are suitable for administration.

The pharmaceutical compositions may be, for example, compositions for enteral, such as oral, rectal, aerosol inhalation or nasal administration, compositions for parenteral, such as intravenous or subcutaneous administration, or compositions for transdermal administration (e.g. passive or iontophoretic).

Preferably, the pharmaceutical compositions are adapted to oral or parenteral (especially intravenous, intra-arterial or transdermal) administration. Intra-arterial and oral, first and foremost intra-arterial, administration is considered to be of particular importance. Preferably the bisphosphonate active ingredient is in the form of a parenteral, most preferably an intra-arterial form.

The particular mode of administration and the dosage may be selected by the attending physician taking into account the particulars of the patient, especially age, weight, life style, activity level, hormonal status (e.g. post-menopausal) and bone mineral density as appropriate. Most preferably, however, the bisphosphonate is administered intra-arterially into an artery which leads to the site of the newly formed blood vessels.

Thus in particularly preferred embodiments the invention provides:

use of a bisphosphonate in the preparation of a medicament as defined above for the intra-arterial embolic treatment of angiogenesis.

The dosage of the Agents of the Invention may depend on various factors, such as effectiveness and duration of action of the active ingredient, mode of administration, warm-blooded species, and/or sex, age, weight and individual condition of the warm-blooded animal.

Normally the dosage is such that a single dose of the bisphosphonate active ingredient from 0.002 - 3.40 mg/kg, especially 0.01 - 2.40 mg/kg, is administered to a warm-blooded animal weighing approximately 75kg. If desired, this dose may also be taken in several, optionally equal, partial doses.

"mg/kg" means mg drug per kg body weight of the mammal - including man - to be treated.

The dose mentioned above - either administered as a single dose (which is preferred) or in several partial doses - may be repeated, for example once daily, once weekly, once every month, once every three months, once every six months or once a year. In other words, the pharmaceutical compositions may be administered in regimens ranging from continuous daily therapy to intermittent cyclical therapy.

Preferably, the bisphosphonates are administered in doses which are in the same order of magnitude as those used in the treatment of the diseases classically treated with bisphosphonic acid derivatives, such as Paget's disease, tumour-induced hypercalcemia or osteoporosis. In other words, preferably the bisphosphonic acid derivatives are administered in doses which would likewise be therapeutically effective in the treatment of Paget's disease, tumour-induced hypercalcaemia or osteoporosis, i.e. preferably they are administered in doses which would likewise effectively inhibit bone resorption.

Formulations in single dose unit form contain preferably from about 1% to about 90%, and formulations not in single dose unit form contain preferably from about 0.1% to about 20%, of the active ingredient. Single dose unit forms such as capsules, tablets or dragées contain e.g. from about 1mg to about 500mg of the active ingredient.

Pharmaceutical preparations for enteral and parenteral administration are, for example, those in dosage unit forms, such as dragées, tablets or capsules and also ampoules. They are prepared in a manner known *per se,* for example by means of conventional mixing, granulating, confectioning, dissolving or lyophilising processes. For example, pharmaceutical preparations for oral administration can be obtained by combining the active ingredient with solid carriers, where appropriate granulating a resulting mixture, and processing the mixture or granulate, if desired or necessary after the addition of suitable adjuncts, into tablets or dragée cores.

Suitable carriers are especially fillers, such as sugars, for example lactose, saccharose, mannitol or sorbitol, cellulose preparations and/or calcium phosphates, for example tricalcium phosphate or calcium hydrogen phosphate, and also binders, such as starch pastes, using, for example, corn, wheat, rice or potato starch, gelatin, tragacanth, methylcellulose and/or polyvinylpyrrolidone and, if desired, disintegrators, such as the above-mentioned starches, also carboxymethyl starch, crosslinked polyvinylpyrrolidone, agar or alginic acid or a salt thereof, such as sodium alginate. Adjuncts are especially flow-regulating agents and lubricants, for example silicic acid, talc, stearic acid or salts thereof, such as magnesium or calcium stearate, and/or polyethylene glycol. Dragee cores are provided with suitable coatings that may be resistant to gastric juices, there being used, inter alia, concentrated sugar solutions that optionally contain gum arabic, talc, polyvinylpyrrolidone, polyethylene glycol and/or titanium dioxide, or lacquer solutions in suitable organic solvents or solvent mixtures or, to produce coatings that are resistant to gastric juices, solutions of suitable cellulose preparations, such as acetylcellulose phthalate or hydroxypropylmethylcellulose phthalate. Colouring substances or pigments may be added to the tablets or dragee coatings, for example for the purpose of identification or to indicate different doses of active ingredient.

Other orally administrable pharmaceutical preparations are dry-filled capsules made of gelatin, and also soft, sealed capsules made of gelatin and a plasticiser, such as glycerol or sorbitol. The dry-filled capsules may contain the active ingredient in the form of a granulate, for example in admixture with fillers, such as lactose, binders, such as starches, and/or glidants, such as talc or magnesium stearate, and, where appropriate, stabilisers. In soft capsules the active ingredient is preferably dissolved or suspended in suitable liquids, such as fatty oils, paraffin oil or liquid polyethylene glycols, it being possible also for stabilisers to be added.

Parenteral formulations are especially injectable fluids that are effective in various manners, such as intravenously, intramuscularly, intraperitoneally, intranasally, intradermally, subcutaneously or preferably intra-arterially. Such fluids are preferably isotonic aqueous solutions or suspensions which can be prepared before use, for example from lyophilised preparations which contain the active ingredient alone or together with a pharmaceutically acceptable carrier. The pharmaceutical preparations may be sterilised and/or contain adjuncts, for example preservatives, stabilisers, wetting agents and/or emulsifiers, solubilisers, salts for regulating the osmotic pressure and/or buffers.

Suitable formulations for transdermal application include an effective amount of the active ingredient with carrier. Advantageous carriers include absorbable pharmacologically acceptable solvents to assist passage through the skin of the host. Characteristically, transdermal devices are in the form of a bandage comprising a backing member, a reservoir containing the compound optionally with carriers, optionally a rate controlling barrier to deliver the active ingredient of the skin of the host at a controlled and predetermined rate over a prolonged period of time, and means to secure the device to the skin.

The following Examples illustrate the invention described hereinbefore and in relation to Example 5 refer to the accompanying Figures:

Figure 1 which are angiograms of the left bronchial ateries of a breast cancer patient before (a) and after (b) treatment with pamidronate disodium;
Figure 2 which are angiograms of the right bronchial ateries of the same breast cancer patient before (a) and after (b) treatment with pamidronate disodium, and
Figure 3 which are angiograms of the right knee of knee joint of an osteoarthritis patient before (a) and after (b) treatment with pamidronate disodium.

In the following Examples the term "active ingredient" is to be understood as being any one of the bisphosphonic acid derivatives mentioned above as being useful according to the present invention.

## EXAMPLES

Example 1: Capsules containing coated pellets of active ingredient, for example, disodium pamidronate pentahydrate, as active ingredient:

| Core pellet: | |
| --- | --- |
| active ingredient (ground) | 197.3 mg |
| Microcrystalline cellulose (Avicel® PH 105) | 52.7 mg |
| | 250.0 mg |
| + Inner coating: | |
| Cellulose HP-M 603 | 10.0 mg |
| Polyethylene glycol | 2.0 mg |
| Talc | 8.0 mg |
| | 270.0 mg |
| + Gastric juice-resistant outer coating: | |
| Eudragit® L 30 D (solid) | 90.0 mg |
| Triethyl citrate | 21.0 mg |
| Antifoam® AF | 2.0 mg |
| Water | |
| Talc | 7.0 mg |
| | 390.0 mg |

A mixture of disodium pamidronate with Avicel® PH 105 is moistened with water and kneaded, extruded and formed into spheres. The dried pellets are then successively coated in the fluidized bed with an inner coating, consisting of cellulose HP-M 603, polyethylene glycol (PEG) 8000 and talc, and the aqueous gastric juice-resistant coat, consisting of Eudragit® L 30 D, triethyl citrate and Antifoam® AF. The coated pellets are powdered with talc and filled into capsules (capsule size 0) by means of a commercial capsule filling machine, for example Höfliger and Karg.

Example 2: Monolith adhesive transdermal system, containing as active ingredient, for example, 1-hydroxy-2-(imidazol-1-yl)-ethane-1,1-diphosphonic acid:

| Composition: | |
| --- | --- |
| polyisobutylene (PIB) 300 | 5.0 g |
| (Oppanol B1, BASF) | 3.0 g |
| PIB 35000 | |
| (Oppanol B 10, BASF) | 9.0 g |
| PIB 1200000 | |
| (Oppanol B100, BASF) | 43.0 g |
| hydrogenated hydrocarbon resin | |
| (Escorez 5320, Exxon) | 20.0 g |
| 1-dodecylazacycloheptan-2-one | |
| (Azone, Nelson Res., Irvine/CA) | 20.0 g |
| active ingredient | |
| Total | 100.0 g |

Preparation:

The above components are together dissolved in 150 g of special boiling point petroleum fraction 100-125 by rolling on a roller gear bed. The solution is applied to a polyester film (Hostaphan, Kalle) by means of a spreading device using a 300mm doctor blade, giving a coating of about 75 g/m$^2$. After drying (15 minutes at 60°C), a silicone-treated polyester film (thickness 75 mm, Laufenberg) is applied as the peel-off film. The finished systems are punched out in sizes in the wanted form of from 5 to 30cm$^2$ using a punching tool. The complete systems are sealed individually in sachets of aluminised paper.

Example 3: Vial containing 1.0 mg dry, lyophilized 1-hydroxy-2-(imidazol-1-yl)ethane-1,1-diphosphonic acid (mixed sodium salts thereof). After dilution with 1 ml of water, a solution (concentration 1 mg/ml) for i.v. infusion is obtained.

| Composition: | |
| --- | --- |
| active ingredient (free diphosphonic acid) | 1.0 mg |
| mannitol | 46.0 mg |
| Trisodium citrate x 2 H$_2$O        ca. | 3.0 mg |
| water | 1 ml |
| water for injection | 1 ml . |

In 1 ml of water, the active ingredient is titrated with trisodium citrate x 2 H$_2$O to pH 6.0. Then, the mannitol is added and the solution is lyophilized and the lyophilisate filled into a vial.

Example 4: Ampoule containing active ingredient, for instance disodium pamidronate pentahydrate dissolved in water. The solution (concentration 3 mg/ml) is for i.v. infusion after dilution.

| Composition: | |
| --- | --- |
| active ingredient<br>    (△ 5.0 mg of anhydrous active ingredient) | 19.73 mg |
| mannitol | 250 mg |
| water for injection | 5 ml. |

Example 5 Treatment of Patients

A number of patients suffering with cancers and associated metastases and one patient suffering with osteoarthritis are treated with bisphosphonate infusions intra-arterially through arteries leading to the cancer, metastasis or osteoarthritic sites. The cancer, metastasis and osteoarthritic sites are examined using standard angiographic techniques both prior to and after bisphosphonate infusion. In all cases a marked embolic effect on the newly formed capillary and other blood vessels in the region the disease site is observed. The treatment regimes are described in greater detail below.

i)

| Patient ID | 1676 |
| --- | --- |
| Gender | Female |
| Age | 68y 6m |
| Diagnosis | Breast Cancer, multiple lung metastases |
| Therapeutic pedicles | the bilateral bronchial arteries |

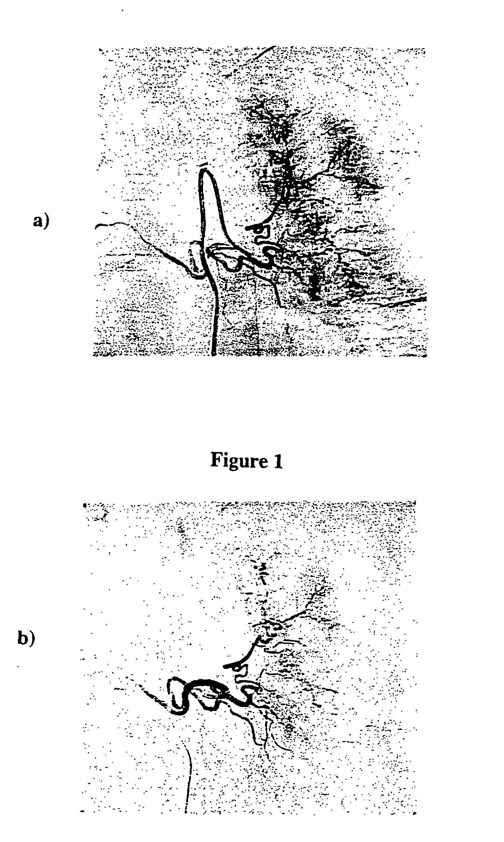

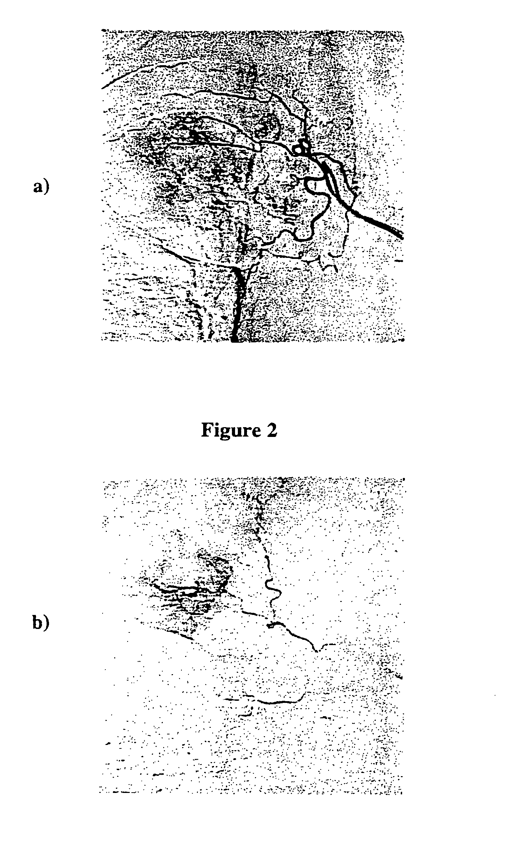

A total of 75 mg of pamidronate disodium (Aredia®) is infused into the bilateral bronchial arteries to obliterate their tumour blushes. Figures 1 and 2 show angiograms of the left (Figure 1) and right (Figure 2) lung areas before (a) and after (b) treatment.

ii)

| Patient ID | 2022 |
|---|---|
| Gender | Female |
| Age | 57y 1m |
| Diagnosis | Breast Cancer, bone metastasis, multiple lung metastases |
| Therapeutic pedicles | 1. bilateral highest intercostal arteries to 12th intercostal artery |
| | 2. bilateral bronchial arteries |
| | 3. lateral internal thoracic artery |
| | 4. vr. lateral thoracic artery |

A total of 75 mg of pamidronate disodium (Aredia®), 100 mg of etoposide, 10 mg of BLM and 500mg of impr as-liprodol. is utilized to obliterate the tumour blushes which are located along these pedicles. (for bone metastases pamidronate is solely used)

iii)

| Patient ID | 1441 |
|---|---|
| Gender | Female |
| Age | 63y 11m |
| Diagnosis | Breast Cancer, multiple bone metastases, multiple hepatic metastases |
| Therapeutic pedicles | 1. bilateral obturator arteries |
| | 2. bilateral L 4/5 to L1 lumbar arteries |
| | 3. right hepatic s 6,7,8 subergemenal hepatic arteries |

A total of 45 mg of pamidronate disodium (Aredia®), 10 mg of ADM (for hepatic) and 500mg of impr as-liprodol emulsion are utilized to obliterate the tumour blushes, which are located along these pedicles (for bone metastases, 45 mg of pamidronate is used on its own and for hepatic metastases 10 mg of ADM with 500mg of impr as-liprodol is additionally utilized).

iv)

| Patient ID | 1840 |
|---|---|
| Gender | Male |
| Age | 43y 11m |
| Diagnosis | Tongue Cancer, colateral vibo, diaphragm metastasis |
| Therapeutic pedicles | 1. the 7,8,9,10th intercostal arteries |
| | 2. lt. ing. phrenia |
| | 3. lt. higher intercostal artery |
| | 4. lt. subscapular artery |

A total of 60 mg of pamidronate disodium (Aredia®) is infused into the above arteries to obliterate their tumour blushes, which are located along these pedicles.

v)

| Patient ID | 1835 |
|---|---|
| Gender | Female |
| Age | 34y 8m |
| Diagnosis | Breast cancer, multiple lung metastases, multiple bone metastases, multiple hepatic metastases |
| Therapeutic pedicles | lt. ileo lumbar, ileo sacral, lateral sacral arteries rt. ileo lumbar, deep ileal orumgles arteries bilat. L4, lt L3, bilat L2 1 thor. arteries, rt. thor. intercostal arteries, bronchial, rt. hepatic artery. |

A total of 90 mg of pamidronate disodium (Aredia®), 100 mg of etoposide, 10 mg of ADM, 6.0 CE of OK-432, and 500mg of impr as-liprodol emulsion is utilized to obliterate the tumour blushes, which are located along these pedicles.

vi)

| Patient ID | 2013 |
|---|---|
| Gender | Female |
| Age | 73y 7m |
| Diagnosis | Right knee joint osteoarthritis (interpreted as an ischemic angiogenic bone disorder) |
| Therapeutic pedicles | 1. right descending genicular artery<br>2. right sular artery |

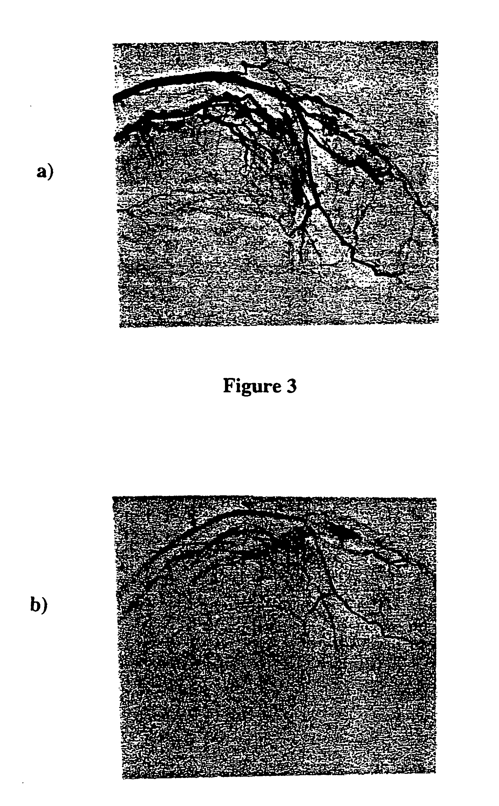

A total of 30 mg of pamidronate disodium (Aredia®) is utilized to obliterate the angiogenic blushes which are located along these pedicles. Angiograms of the area of the right knee joint are shown in Figure 3 before (a) and after (b) treatment.

vii)

| Patient ID | 2026 |
|---|---|
| Gender | Female |
| Age | 49y |
| Diagnosis | Breast Cancer, Thoracic spine, bone metastases |
| Therapeutic pedicles | 1. bilateral highest intercostal arteries<br>2. bilateral 1-4 lumbar arteries<br>3. median sacral artery |

A total of 30 mg of pamidronate disodium (Aredia®) is infused into the tumour blushes to obliterate them.

viii)

| Patient ID | 1985 |
|---|---|
| Gender | Female |
| Age | 49y 8m |
| Diagnosis | Breast Cancer, multiple bone metastases After BCT |
| Therapeutic pedicles | 1. The bilateral highest intercostal to 12th intercostal arteries<br>2.The bilateral lumbar arteries (L1 to L4)<br>3. the median sacral artery |

A total of 90 mg of pamidronate disodium (Aredia®), is utilized to obliterate the tumour blushes, which are located along these pedicles.

ix)

| Patient ID | 1063 |
|---|---|
| Gender | Male |
| Age | 80y 10m |
| Diagnosis | Lung Cancer, squamous cell cancer |
| Therapeutic pedicles | 1. right highest intercostal artery<br>2. common trunk of the bilateral bronchial arteries |

A total of 45 mg of pamidronate disodium (Aredia®), is solely infused into the tumour to obliterate the tumour blushes of the feeder pedicles.

Example 6 Effect of zoledronic acid on angiogenesis induced by growth factor impregnated, subcutaneous implants in mice

**Methods**

**Animals**

Female mice (Tiflbm:MAG ) weighing 17 to 20 g were used. They were identified via ear markings and kept in groups (6 animals per cage) under normal conditions and observed daily. Six mice were used per treatment group in each experiment. All experiments were performed at least twice.

**Preparation of chamber**

Porous tissue chambers made of perfluoro-alkoxy-Teflon (Teflon®-PFA, 21 mm x 8 mm diameter, 550 $\mu$l volume) and perforated with 80 regularly spaced 0.8 mm holes were used. Both ends were sealed with removable caps of the same material. Four to six chambers were inserted in a silicon tube (12 mm diameter). Both ends of the tube were then sealed with silicon rubber and 24 h later the tube containing the chambers was sterilized by autoclaving (121°C, 15 min).

**Growth factors**

The chambers were filled (total volume 0.5 ml) under sterile conditions, while still contained within the silicon tube, with agar 0.8% w/v (BBL® Nr. 11849, Becton Dickinson, Meylan, France) containing 20 U/ml heparin (Novo Nordisk A/S, 2880 Bagsvaerd, Denmark) and with or without growth factor (human VEGF 2 $\mu$g/ml or human bFGF 0.3 $\mu$g/ml). The agar solution was maintained at 42°C prior to the filling procedure.

**Chamber implantation**

Anaesthesia was induced in the mice by inhalation of 3% isoflurane (Forene®, Abbott AG, Cham, Switzerland) in oxygen. The chamber was implanted under aseptic conditions through a small incision on the back of the animal. The skin incision was closed by wound clips (Autoclip 9 mm, Clay Adams).

**Chamber removal**

Five days after implantation, animals were anaesthetized (3% isoflurane) and sacrificed using an overdose of pentobarbitone (210 mg/kg i.p., Vetanarcol, Veterinaria AG, Zürich, Switzerland). Chambers were then taken out of the animal, the vascularized fibrous tissue that had formed around each implant was carefully removed, weighed and the total amount of blood was quantified by colorimetric determination of the haemoglobin concentration.

**Quantification of the angiogenic response**

After addition of 2 ml of distilled water, tissue samples were homogenised for 1 min at 24000 rpm (Ultra Turrax T25). The samples were then centrifuged for 1 h at 7000 rpm. The supernatant was filtered through a 0.45 $\mu$m GHP syringe filter (Acrodisc® GF, Gelman Sciences, Ann Arbor, Michigan, USA) to avoid fat contamination. The amount of haemoglobin present in the filtrate was determined by spectrophotometric analysis at 540 nm using the Drabkin reagent kit (Sigma haemoglobin #525, Sigma Chemical Co. Ltd., Poole, Dorset, England). An aliquot of the filtrate (100 $\mu$l) was added to 1 ml of Drabkin's solution and the mixture incubated for 15 min at room temperature. The absorbance at 540 nm (Uvikon 810 P) is proportional to the haemoglobin concentration. The haemoglobin measurements were then converted to a blood volume measurement ($\mu$l) using a calibration curve previously obtained with whole blood samples of different volumes from a donor mouse.

**Drug treatment**

Zoledronic acid administration was started one day before implantation of the chambers and the chambers were removed 24 h after the last dose, 5 days after implantation. Animals were housed in groups for the duration of an experiment. Zoledronic acid was given in doses of 1, 10 and 100 $\mu$g/kg/day s.c. (dose volume 25 ml/kg) with injection given at rotating sites. The compound was first dissolved in distilled water and then diluted with distilled water containing mannitol (final concentration: 5% mannitol). Control animals received vehicle alone. Each experiment was performed at least twice and data then pooled for the final evaluation.

**Calculations and statistics**

The percentage inhibition of the angiogenic response (increase in tissue weight or total blood) was calculated in individual animals as follows:

$$(A-B)/(C-D) \times 100.$$

A = weight (or blood volume) of the tissue from a drug-treated mouse with a chamber containing growth factor.

B = mean weight (or blood volume) of the tissue from the group of drug-treated mice with chambers not containing growth factor.

C = mean weight (or blood volume) of the tissue from the group of vehicle-treated mice with chambers containing growth factor.

D = mean background weight (or blood volume) of the tissue from the group of vehicle-treated mice with chambers not containing growth factor.

The statistical comparisons between groups (compound-treated versus non-treated animals) were performed on the absolute values for tissue weight and blood volume using the Mann-Whitney rank sum test (SigmaStat 2.0 software, Jandel Scientific, Germany). The significance level was set at $p < 0.05$.

**Results**

Zoledronic acid dose-dependently inhibited the angiogenic response induced by bFGF, as measured by weight and blood content, with approximate $IC_{50}$ values of 2.5 and 3.1 μg/kg, respectively. At the highest dose tested (100 μg/kg/day), there was a significant inhibition of the VEGF-induced increase in blood content but not in weight.
These studies demonstrate that zoledronic acid inhibits bFGF but not VEGF induced angiogenesis in a dose range that shows therapeutic effects on bone turnover in several disease animal models. At higher doses a partial inhibition of the VEGF response was also observed. Thus, zoledronic acid seems to have some selectivity for inhibition of the bFGF-mediated response.

Example 7 Effects of Zoledronate on cell proliferation and migration in vitro

**Methods**

**Test compounds and solutions**

The studies described in this report were performed with the hydrated disodium salt of zoledronate. Stock solutions of zoledronate (10 mM) and EDTA (5 mM) were prepared in PBS. Stock solutions were then diluted in the optimal medium for each cell line.

**Cells and cell culture conditions**

Human umbilical vein endothelial cells (HUVEC) were obtained from Promo Cell (BioConcept AG, Allschwil, Switzerland). The human tumour cell line, A431 (epithelial carcinoma) used in this study was obtained from the American Type Culture Collection (ATCC, Rockville, MD, USA). All cell lines were cultivated *in vitro* according to the recommendations of the supplier.

**Endothelial cell proliferation assay**

As a test of the ability of zoledronate to inhibit a functional response to VEGF, an endothelial cell proliferation assay, based on BrdU incorporation was used (Biotrak Cell Proliferation Elisa System V.2, Amersham, England). To test the specificity of the response, the effects of zoledronate and EDTA (as a control for the ion chelating effects of zoledronate) on bFGF- and serum-induced proliferation of HUVEC were also tested. Subconfluent HUVEC were seeded at a density of $5 \times 10^3$ cells per well into 96-well plates coated with 1.5% gelatin and then incubated at 37°C and 5% $CO_2$ in growth medium (Endothelial Cell Growth medium, PromoCell Nr. C-22110, BioConcept AG, Allschwil, Switzerland). After 24 h, the growth medium was replaced by basal medium (Endothelial Cell Basal medium, PromoCell Nr. C-22210, BioConcept AG, Allschwil, Switzerland) containing human $VEGF_{165}$ (10 ng/ml), bFGF (0.5 ng/ml) or 5% FCS, in the presence or absence of test compound. As a control, wells without growth factor were also included. After 24 h of incubation, BrdU labelling solution was added and cells incubated a further 24 h before fixation, blocking and addition of peroxidase-labelled anti-BrdU antibody. Bound antibody was then detected using 3,3'5,5'-tetramethylbenzidine substrate, which forms a coloured reaction product that is quantified spectrophotometrically at 450 nm.

**Tumour cell proliferation assay**

To further test for the specificity of zoledronate and EDTA, their effects on the proliferation of a human tumour cell line (A431, epithelial carcinoma) were tested. Cells were seeded at $1.5 \times 10^3$ cells/well into 96-well plates and incubated overnight. Test compound was then added in serial dilutions and the plates were then incubated for 3 days, after which the cells were fixed with 3.3% v/v glutaraldehyde, washed with water and stained with 0.05% w/v methylene blue. After washing, the dye was eluted with 3% v/v HCl and the optical density measured at 665 nm with a Dynatech 7000 spectrophotometer. The percentage reduction in cell growth of cells exposed to compound as compared to controls was determined by a computerized system using the formula (OD test - OD start) / (OD control - OD start) x 100. The $IC_{50}$ was defined as the drug concentration which leads to a 50% reduction in the number of cells per well compared to control cultures (100%) at the end of the incubation period.

**Endothelial cell migration assay**

As a test of the ability of zoledronate to inhibit a functional response to VEGF, an endothelial cell migration assay was used. Plates (24-well) were coated with 1.5% gelatin and fitted with circular fences as a barrier to prevent cells from growing in the center of the well. Subconfluent HUVEC were seeded into the outer area at a density of $1 \times 10^5$ cells per well and then incubated at 37°C and 5% $CO_2$ in growth medium (Endothelial Cell Growth medium, PromoCell Nr. C-22110, BioConcept AG, Allschwil, Switzerland). After 24 h, the fences were removed and the growth medium was replaced by basal medium (Endothelial Cell Basal medium, PromoCell Nr. C-22210, BioConcept AG, Allschwil, Switzerland) containing human $VEGF_{165}$ (10 ng/ml) or 5% FCS, in the presence or absence of test compound. To inhibit the cell proliferation, 50 µg/ml fluorouracil (Roche, Basel, Switzerland) was added. As a control, wells without growth factor were also included. After 60-70 h of incubation, the cells were fixed and stained with Diff-Quik (Dade Behring AG., Nr. 130832, Düdingen, Switzerland). The 24-well plates were placed under a binocular microscope and the number of migrated cells was counted, using the software KS-400 (Carl Zeiss Jena, Jena, Germany) and a specially designed macro (migration.mcr).

**Statistics**

Data were analyzed by one-way analysis of variance followed by Dunnett's test to assess the significance of differences between the zoledronate or EDTA treated groups and the control. The Bonferroni test was used to assess the significance of differences between each pair of zoledronate and EDTA treated groups at a specific concentration. All analyses were performed with the Instat® software (GraphPad Inc., San Diego, USA).

**Results**

Zoledronate dose-dependently inhibited serum-induced HUVEC proliferation with an $IC_{50}$ value of $4.1 \pm 0.6$ µM. It also had a similar effect on the proliferation induced by VEGF ($IC_{50}$ : $6.9 \pm 0.4$ µM) and by bFGF ($IC_{50}$: $4.2 \pm 0.4$ µM). The inhibitory effect of zoledronate on proliferation was significantly different from control at the higher concentrations. For all stimuli, changes in cell morphology were observed at the highest concentration of zoledronate tested (30 µM).

As bisphosphonates chelate divalent cations, EDTA was used as a control in parallel experiments. EDTA at 30 µM inhibited HUVEC proliferation induced by FCS, VEGF and bFGF by 23.7%, 55.6% and 49.5%, respectively. For all stimuli, the effect of EDTA was less than that of zoledronate, it did not reach statistical significance for serum-induced proliferation within the concentration range tested, whereas it did with VEGF and bFGF stimulation at EDTA concentrations of 3 µM and above. For stimulation with serum or bFGF, the inhibitory effect of zoledronate was significantly greater than that of EDTA at concentrations of 3 µM and above, but with VEGF stimulation only the 30 µM values were significantly different.

The proliferation of the human tumour cell line A431 was inhibited by zoledronate with an $IC_{50}$ of 1.35 µM.

EDTA had no significant effect on A431 proliferation in the tested dose range although a slight inhibition (8.8%) was apparent at 30 µM.

Zoledronate at concentrations of up to 10 µM stimulated HUVEC migration in basal medium as well as in medium containing serum or VEGF. At 30 µM migration was completely inhibited and a change in cell morphology was observed. EDTA had no consistent effects on migration.

These studies demonstrate that zoledronate inhibits proliferation of human endothelial cells induced by serum, VEGF and bFGF.

## Claims

1. Use of a bisphosphonate in the preparation of a medicament for the treatment of myocardial ischemia, osteoarthritis, psoriasis, haemangioblastoma, haemangioma or pain, wherein the bisphosphonate acts as an angiogenesis inhibiting or reversing agent and is selected from the following compounds or a pharmaceutically acceptable salt thereof, or any hydrate thereof: 3-amino-1-hydroxyprogane-1,1-diphosphonic acid (pamidronic acid), e.g. pamidronate (APD); 3-(N,N-dimethylamino)-1-hydroxypropane-1,1-diphosphonic acid, e.g. dimethyl-APD; 4-amino-1-hydroxybutane-1,1-diphosphonic acid (alendronic acid), e.g. alendronate; 1-hydroxy-ethidene-bisphosphonic acid, e.g. etidronate; 1-hydroxy-3-(methylpentylamino)-propylidene-bisphosphonic acid, ibandronic acid, e.g. ibandronate; 6-amino-1-hydroxyhexane-1,1-diphosphonic acid, e.g. amino-hexyl-BP; 3-(N-methyl-N-n-pentylamino)-1-hydroxypropane-1,1-diphosphonic acid, e.g. methyl-pentyl-APD (= BM 21.0955); 1-hydroxy-2-(imidazol-1-yl) ethane-1,1-diphosphonic acid, e.g. zoledronic acid; 1-hydroxy-2-(3-pyridyl)ethane-1,1-diphosphonic acid (risedronic acid), e.g. risedronate, including N-methyl pyridinium salts thereof, for example N-methyl pyridinium iodides such as NE-10244 or NE-10446; 1-(4-chlorophenylthio)methane-1,1-diphosphonic acid (tiludronic acid), e.g. tiludronate; 3-[N-(2-phenylthioethyl)-N-methylamino]-1-hydroxypropane-1,1-diphosphonic acid; 1-hydroxy-3-(pyrrolidin-1-yl)propane-1,1-diphosphonic acid, e.g. EB 1053 (Leo); 1-(N-phenylaminothiocarbonyl)methane-1,1-diphosphonic acid, e,g, FR 78844 (Fujisawa); 5-benzoyl-3,4-dihydro-2H-pyrazole-3,3-diphosphonic acid tetraethyl ester, e.g. U-81581 (Upjohn); 1-hydroxy-2-(imidazo[1,2-a]pyridin-3-yl)ethane-1,1-diphosphonic acid, e.g. YM 529; and 1,1-dichloromethane-1,1-diphosphonic acid (clodronic acid), e.g. clodronate.

2. A use according to claim 1, in which the bisphosphonate is used in the preparation of a medicament for the embolic treatment of angiogenesis.

3. A use according to claim 1, in which the bisphosphonate is used in the preparation of a medicament for the prophylactic or preventative treatment of angiogenesis.

4. A use according to claim 1, in which the bisphosphonate is used in the preparation of a medicament for the treatment of angiogenesis in a patient suffering from myocardial ischemia or osteoarthritis.

5. A use according to claim 1, in which the bisphosphonate is pamidronic acid or zoledronic acid, or a pharmaceutically acceptable salt thereof, or any hydrate thereof.

6. A use according to claim 1, in which the bisphosphonate is used in the preparation of a medicament for the intra-arterial embolic treatment of angiogenesis.

7. A use according to claim 6, in which the bisphosphonate is pamidronic acid or zoledronic acid, or a pharmaceutically acceptable salt thereof, or any hydrate thereof.

## Patentansprüche

1. Verwendung eines Bisphosphonats zur Herstellung eines Arzneimittels zur Behandlung von Myokardischämie, Osteoarthritis, Psoriasis, Hämangioblastom, Hämangiom oder Schmerz, worin das Bisphosphonat als Angiogenese hemmendes oder verhinderndes Mittel wirkt und aus den folgenden Verbindungen oder einem pharmazeutisch annehmbaren Salzes hiervon oder einem Hydrat hiervon ausgewählt ist: 3-Amino-1-hydroxypropan-1,1-diphosphonsäure (Pamidronsäure), beispielsweise Pamidronat (APD), 3-(N,N-Dimethylamino)-1-hydroxypropan-1,1-diphosphonsäure, beispielsweise Dimethyl-APD, 4-Amino-1-hydroxybutan-1,1-diphosphonsäure (Alendronsäure), beispielsweise Alendronat, 1-Hydroxyethidenbisphosphonsäure, beispielsweise Etidronat, 1-Hydroxy-3-(methylpentylamino)propylidenbisphosphonsäure, Ibandronsäure, beispielsweise Ibandronat, 6-Amino-1-hydroxyhexan-1,1-diphosphonsäure, beispielsweise Aminohexyl-BP, 3-(N-Methyl-N-n-pentylamino)-1-hydroxypropan-1,1-diphosphonsäure, beispielsweise Methylpentyl-APD (= BM 21.0955), 1-Hydroxy-2-(imidazol-1-yl)ethan-1,1-diphosphonsäure, beispielsweise Zoledronsäure, 1-Hydroxy-2-(3-pyridyl)ethan-1,1-diphosphonsäure (Risedronsäure), beispielsweise Risedronat, einschließlich N-Methylpyridiniumsalze hiervon, beispielsweise N-Methylpyridiumiodide, wie NE-10244 oder NE-10446, 1-(4-Chlorphenylthio)-methan-1,1-diphopshonsäure (Tiludronsäure) beispielsweise Tiludronat, 3-[N-(2-Phenylthioethyl)-N-methylamino]-1-hydroxypropan-1,1-diphosphonsäure, 1-Hydroxy-3-(pyrrolidin-1-yl)-propan-1,1-diphosphonsäure, beispielsweise EB 1053 (Leo), 1-(N-Phenylaminothiocarbonyl)methan-1,1-diphosphonsäure, beispielsweise FR 78844 (Fujisawa), 5-Benzoyl-3,4-dihydro-2H-pyrazol-3,3-diphosphonsäuretetraethylester, beispielsweise U 81581 (Upjohn), 1-Hydroxy-2-(imidazo[1,2-a]pyridin-3-yl)ethan-1,1-diphosphonsäure, beispielsweise YM 529 und 1,1-Dichlormethan-1,1-diphosphonsäure (Clodronsäure), beispielsweise Clodronat.

2. Verwendung nach Anspruch 1, worin das Bisphosphonat zu Herstellung eines Arzneimittels zur embolischen Behandlung der Angiogenese verwendet wird.

3. Verwendung nach Anspruch 1, worin das Bisphosphonat zur Herstellung eines Arzneimittels zur prophylaktischen oder präventiven Behandlung der Angiogenese verwendet wird.

4. Verwendung nach Anspruch 1, worin das Bisphosphonat zur Herstellung eines Arzneimittels zur Behandlung der Angiogenese bei einem Patienten verwendet wird, der an einer Myokardischämie oder Osteoarthritis leidet.

5. Verwendung nach Anspruch 1, worin das Bisphosphonat Pamidronsäure oder Zoledronsäure oder ein pharmazeutisch annehmbares Salz hiervon oder ein Hydrat hiervon ist.

6. Verwendung nach Anspruch 1, worin das Bisphosphonat zur Herstellung eines Arzneimittels zur intraarteriellen, embolischen Behandlung der Angiogenese verwendet wird.

7. Verwendung nach Anspruch 6, worin das Bisphosphonat Pamidronsäure oder Zoledronsäure ist oder ein pharmazeutisch annehmbares Salz hiervon oder ein Hydrat hiervon.

**Revendications**

1. Utilisation d'un bisphosphonate dans la préparation d'un médicament pour le traitement de l'ischémie du myocarde, de l'ostéo-arthrite, du psoriasis, de l'haemangioblastome, de l'hémangiome ou de la douleur, **caractérisée en ce que** le bisphosphonate agit en tant qu'agent d'inhibition ou d'inversion de l'angiogenèse et **en ce qu'**il est sélectionné à partir des composés suivants ou de leur sel pharmaceutiquement acceptable, ou d'un de leurs hydrates : acide 3-amino-1-hydroxypropane-1,1-diphosphonique (acide pamidronique), p.ex. pamidronate (APD); acide 3-(N,N-diméthylamino)-1-hydroxypropane-1,1-diphosphonique, p.ex. diméthyl-APD ; acide 4-amino-1-hydroxybutane-1,1-diphosphonique (acide alendronique), p.ex. alendronate ; acide 1-hydroxy-éthidène-bisphosphonique, p. ex. étidronate ; acide 1-hydroxy-(3-méthylpentylamino)-propylidène-bisphosphonique, acide ibandronique, p.ex. ibandronate ; acide 6-amino-1-hydroxyhexane-1,1-diphosphonique, p.ex. amino-hexyl-BP; acide 3-(N-méthyl-N-n-pentylamino)-1-hydroxypropane-1,1-diphosphonique, p.ex. méthyl-pentyl-APD (= BM 21,0955) ; acide 1-hydroxy-2-(imidazol-1-yl)éthane-1,1-diphosphonique, p.ex. acide zolédronique ; acide 1-hydroxy-2-(3-pyridyl)éthane-1,1-diphosphonique (acide risedronique), p.ex. risedronate, y compris leurs sels N-méthyle pyridinium, par exemple les iodures N-méthyle pyridinium tels que NE-10244 ou NE 10446; acide 1-(4-chlorophénylthio)méthane-1,1-diphosphonique (acide tiludronique), p.ex. tiludronate ; acide 3-[N-(2-phénylthioéthyl)-N-méthylamino]-1-hydroxypropane-1,1-diphosphonique; acide 1-hydroxy-(3-pyrrolidin-1-yl)propane-1,1-diphosphonique, p.ex. EB 1053 (Leo) ; acide 1-(N-phénylaminothiocarbonyl)méthane-1,1-diphosphonique, p.ex. FR 78844 (Fujisawa) ; ester tétraéthylique d'acide 5-benzoyl-3,4-dihydro-2H-pyrazole-3,3-diphosphonique, p.ex. U-81581 (Upjohn); acide 1-hydroxy-2-(imidazo[1,2-a]pyridin-3-yl)éthane-1,1-diphosphonique, p.ex. YM 529 et acide 1,1-dichlorométhane-1,1-diphosphonique (acide clodronique), p.ex. clodronate.

2. Utilisation selon la revendication 1, **caractérisée en ce que** le bisphosphonate est employé dans la préparation d'un médicament pour le traitement embolique de l'angiogenèse.

3. Utilisation selon la revendication 1, **caractérisée en ce que** le bisphosphonate est employé dans la préparation d'un médicament pour le traitement prophylactique ou préventif de l'angiogenèse.

4. Utilisation selon la revendication 1, **caractérisée en ce que** le bisphosphonate est employé dans la préparation d'un médicament pour le traitement de l'angiogenèse chez un patient souffrant d'ischémie du myocarde ou d'ostéoarthrite.

5. Utilisation selon la revendication 1, **caractérisée en ce que** le bisphosphonate est un acide pamidronique ou un acide zoledronique ou un de leurs sels pharmaceutiquement adéquats ou un de leurs hydrates.

6. Utilisation selon la revendication 1, **caractérisée en ce que** le bisphosphonate est employé dans la préparation d'un médicament pour le traitement embolique intra-artériel de l'angiogenèse.

7. Utilisation selon la revendication 6, **caractérisée en ce que** le bisphosphonate est un acide pamidronique ou un acide zoledronique ou un de leurs sels pharmaceutiquement adéquats ou un de leurs hydrates.

a)

**Figure 1**

b)

a)

**Figure 2**

b)

a)

**Figure 3**

b)